(19)

Europäisches
Patentamt
European
Patent Office
Office européen
des brevets

(11) **EP 4 295 777 A1**

(12) **EUROPEAN PATENT APPLICATION**

(43) Date of publication:
    **27.12.2023   Bulletin 2023/52**

(21) Application number: **22193386.4**

(22) Date of filing: **01.09.2022**

(51) International Patent Classification (IPC):
    **A61B 6/03** *(2006.01)*      **A61B 6/00** *(2006.01)*
    **G06T 11/00** *(2006.01)*

(52) Cooperative Patent Classification (CPC):
    **A61B 6/037; A61B 6/5205; G06T 11/003;**
    **G06T 11/008**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **KH MA MD TN**

(30) Priority:   **23.06.2022   US 202263354746 P**

(71) Applicant: **Koninklijke Philips N.V.**
    **5656 AG Eindhoven (NL)**

(72) Inventors:
    • **GOEDICKE, Andreas Georg**
      **Eindhoven (NL)**

    • **WUELKER, Christian**
      **Eindhoven (NL)**
    • **PERKINS, Amy**
      **5656AG Eindhoven (NL)**
    • **SALOMON, Andre Frank**
      **5656AG Eindhoven (NL)**
    • **ZHANG, Bin**
      **Eindhoven (NL)**
    • **GRASS, Michael**
      **Eindhoven (NL)**

(74) Representative: **Philips Intellectual Property &
    Standards
    High Tech Campus 52
    5656 AG Eindhoven (NL)**

(54)   **DEVICE, SYSTEM AND METHOD FOR PROCESSING PET IMAGING DATA**

(57)      The present invention relates to the field of medical imaging, in particular to Positron Emission Tomography (PET), PET image reconstruction. A device (20) for processing PET imaging data is presented, wherein the device is adapted to perform the steps of: obtaining PET imaging data (201) (S101); dividing the PET imaging data into subsets (212) (S102); reconstructing a set of surrogate PET images (214) based on the subsets (212) (S103), wherein a surrogate PET image is reconstructed for each of the subsets; determining a variance (216) between images of the set of surrogate PET images (S104); determining a confidence level measure (220, 222) based on an expected variance and the determined variance between images of the set of surrogate PET images (S 105). Further, a corresponding method, computer program and system are provided.

FIG.3

EP 4 295 777 A1

**Description**

FIELD OF THE INVENTION

The present invention relates to the field of medical imaging, in particular to Positron Emission Tomography (PET), PET image reconstruction and to devices, systems and methods for supporting a clinician in interpreting a reconstructed output image.

BACKGROUND OF THE INVENTION

Positron emission tomography (PET) is a medical imaging technique, in particular a medical scintillography technique, used in nuclear medicine and clinical oncology. The concentrations of a tracer can indicate a tissue metabolic activity, for example corresponding to a regional glucose uptake that can be indicative of cancerous tissue.

PET imaging tries to reveal the spatial distribution of specific biological (e.g. metabolic or signaling) processes inside the body by observing radiation emitted by radiotracers designed to target these processes. In the subsequent diagnosis, deviations from the expected spatial distribution pattern are linked to malignancies, such as oncological, neurological, or craniological diseases. The diagnostic quality is determined by numerous factors: physiological, such as the binding characteristics of the applied radiotracer, physical, such as the creation characteristics of the measured radiation, and scanner design-related affecting the overall sensitivity of radiation detection. Constant effort is taken on the device manufacturer side to improve the scanner hard- and software, as well as on the radiopharmaceutical side to come up with new, preferably highly specific, tracers, in order to further extend the PET application spectrum.

One of the main intrinsic limitations in PET is the statistical nature of the detected radiation in combination with the impact, and resulting alteration, caused by its passage through the body on its way to the detection unit. While the latter effects are well understood and may even be patient-individually compensated using advanced physical model-based techniques, the only way to reduce the signal noise is to increase the amount of data measured, either by extending the scan time, by increasing the administered tracer dose or by increasing the scanner sensitivity. There are limits to all these approaches, either from an ethical, legal, for example due to radiation safety, or technological standpoint. Accordingly, the problem of drawing conclusions from noisy data remains.

WO 2018/220182 A1 discloses systems and methods to provide confidence values as a measure of quantitative assurance for iteratively reconstructed images in emission tomography. The teachings therein focus on iterative image reconstruction. Iterative image reconstruction in emission imaging (e.g. positron emission tomography, PET, or single photon emission computed tomography, SPECT) has become an established approach for reconstructing medical diagnostic quality images. In iterative reconstruction, different structures may have different speed of convergence. It often takes more iterations for small structures (i.e., those represented by high frequencies) to converge. However, when more iterations are used, noise may increase as well. Therefore, clinical reconstruction protocols usually use a predefined number of iterations and subsets that have been optimized for the general case of clinical applications. However, when reconstruction finishes, some small lesions may not be fully converged, i.e., their values would change significantly if further iterations were to be performed. This leads to quantitative uncertainty of such lesions. For example, a certain (hypothetical) lesion would have a standardized uptake value (SUV) of 5.0 at full convergence, but when the reconstruction with certain non-specifically optimized number of iterations is finished, it is not converged yet, and the reconstructed image only shows SUV of 2.5. The size of the ROI, point spread function (PSF) of the device and the number of acquired counts in the ROI also directly affect the confidence of the reported SUV value. In view of the foregoing, when a physician is presented with the final reconstructed image, there may be limited assurance that the values in the image are quantitatively accurate for all the regions/objects in the image whenever iterative reconstruction is involved. Typically, the physician is provided with little or no information as to the reliability (or lack thereof) of quantitative values. Nonetheless, PET or SPECT images are increasingly interpreted quantitatively for treatment-determinative clinical purposes such as early detection of the success or failure of radiation therapy, chemotherapy, or other oncology therapy regimens. Physicians desire to draw clinical conclusions from small changes in successive imaging examinations. For example, a change in tumor size (e.g., a change in physical size, a change in a maximum SUV value of the tumor, and the like) of a few percent may be interpreted by the physician as indicative of therapeutic efficacy leading to continuation of the therapy - but such a small change might instead be due to incomplete convergence, partial volume effect (PVE), and/or statistical fluctuations.

WO 2018/220182 A1 suggests an image reconstruction method comprising inter alia the steps of generating at least one reconstructed image; delineating one or more contours of the at least one reconstructed image to determine a region of interest (ROI) of the at least one reconstructed image; computing at least one quality metric value of the ROI of the at least one reconstructed image; and displaying, on the display device, the at least one quality metric value and the at least one reconstructed image showing the ROI.

SUMMARY OF THE INVENTION

It is an object of the present invention to provide a further improved device, system and method to be used in supporting a clinician in interpreting a reconstructed output image. In particular, it would be desirable to determine information how much a presented PET image feature is supported by the (stability of the) underlying information in the measured data.

In a first aspect of the present disclosure, a device for processing PET imaging data is presented. The device is adapted to perform the steps of:

- obtaining PET imaging data;
- dividing the PET imaging data into subsets;
- reconstructing a set of surrogate PET images based on the subsets, wherein a surrogate PET image is (separately) reconstructed for each of the subsets;
- determining a variance between images of the set of surrogate PET images;
- determining a confidence level measure based on an expected variance and the determined variance between images of the set of surrogate PET images

In a further aspect of the present disclosure, a positron emission tomography, PET, system is presented, the system comprising: a positron emission tomography, PET, scanner adapted to acquire PET imaging data; and a device for processing PET imaging data as described above.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, PET system, computer program and medium can have similar and/or identical preferred embodiments as the claimed device, in particular as defined in the dependent claims and as disclosed herein.

The inventors recognized that in PET image reconstruction, signal noise may be transformed to, in particular clustered, image noise, affecting the diagnostic quality especially for small or weakly binding anatomical structures, e.g., small tumors, lymph nodes, etc. Depending on the local noise level in the image, true structures can easily be missed (false-negative finding), and artificial structures from clustered noise can be easily misinterpreted as relevant (false-positive) findings. Different approaches may be pursued in order to reduce PET image noise either as post-processing of the reconstructed image or as an intra-iterative filtering step during the reconstruction. It was recognized that, the latter methods, such as using spatial noise-regularization priors, bear the potential to better preserve, especially small, anatomical features. However, which image features are actually preserved and which are suppressed may depend on the chosen prior characteristics, as well as on the pre-sorting of the detected signal as input to the image reconstruction. Depending on how noise events are randomly clustered in the list-mode data subsets, e.g. in OS-EM-type reconstruction algorithms, noise-based artificial spots might form and be preserved, or might be suppressed.

In addition, changes in the local tracer concentration, such as delayed or late accumulation effects, during the image acquisition might not fulfil the underlying assumption for, in particular non-dynamic, PET that changes in the observed average number of detected counts for a given anatomical region are dominated by the decay of the involved radioisotope. Again, depending on the level of such dynamic processes, the reliability of quantitative tracer concentration measures based on the final PET image might be significantly reduced by the way this increased variance is distributed over the PET subsets and adjacent scan positions. The inventors recognized that one or more of these effects might be especially relevant for modern, in particular prior-based, regularized reconstruction methods.

Separating artefacts, such as noise-founded artefacts, from relevant image features and also estimating the relative contribution of physiological effects on the measurement result often heavily depend on the experience-level of the nuclear medicine physician. Moreover, even an experienced physician considering contextual information may be faced with the problem of separating artefacts and relevant image features in the region of interest.

The present invention is based on the idea to provide an easy to implement yet effective approach to providing additional information on how much a presented PET image feature is supported by the underlying information in the measured data, in particular by the stability of the underlying information in the measured data. The result can for example be used to draw the clinician's attention to suspicious anatomical regions where special attention in the image interpretation is demanded, or to better characterize an image finding, or to directly adapt the PET image information displayed to the clinician. It is suggested to determine a confidence level or uncertainty level measure for features that can be observed in a reconstructed image. Based thereon a visualization of an uncertainty of one or more image regions in reconstructed PET images, i.e., information on the reliability of whether or not a feature is a lesion or an artifact, can be provided to a clinician. Similarly, there may also be a region without a feature but with high uncertainty, increasing the risk for false negatives.

Briefly summarized, the idea is to divide the obtained PET imaging data into a plurality of subsets. Measured events, also referred to counts herein, in the PET imaging data can be divided into a number of subsets. Reconstruction is then performed separately on each of these subsets. Rather than using the entire obtained PET data to reconstruct only one PET image, a surrogate PET image is separately reconstructed for each of the subsets. This results in a plurality or set of surrogate PET images that can be compared with one another. It is for example possible to compare the reconstructed images on a per-voxel basis or using groups of voxels. A variance between images of the set of surrogate PET images can be determined. This allows to provide a variance map indicative of a variation of voxels (voxel values/intensities) between images of the set of surrogate PET images. A strong variation between images of the set of surrogate PET images indicates a high uncertainty or low confidence level in a respective spatial region in an image reconstructed using the (entire) PET imaging data. In this way, it is possible to quantify a statistical uncertainty in the measured data in a spatially resolved way.

An advantage of the proposed approach can also be that it can be used even if the assumption of Poisson statistics does not hold, e.g. in case of patient motion of a non-stationary (dynamic) tracer distribution.

As used herein, the term obtaining PET imaging data may refer to receiving or retrieving PET imaging data. The data may be obtained directly from a PET imaging device adapted to (physically) acquire PET imaging data but may also be obtained from a data storage such as a memory or database. As used herein, PET imaging data can refer to PET imaging data acquired during the same or single imaging session. The PET imaging data can for example be PET list-mode data or sinogram data. As used herein, a surrogate PET image may refer to a PET image reconstructed from a subset of the PET imaging data.

Dividing the PET imaging data into subsets can comprise dividing the measured counts of the PET imaging data into a number of subsets, each subset having fewer counts than the measured counts of the PET imaging data. As used herein, an event or count may refer to an acquired positron emission event in the PET imaging data. The total number of counts may be given by N and each subset has fewer counts Nb<N than the measured counts of the PET imaging data. The number of counts may differ between subsets. The subsets can be disjunctive subsets, wherein each count belongs to exactly one subset. However, it is also possible that one or more subsets have an overlap, wherein one or more counts may be part of one or more different subsets. This allows more flexibility in creating the subsets. An advantage of having overlapping subsets is that larger numbers of counts can reduce statistical fluctuations. Nevertheless, the division of the PET imaging data into subsets stills allows to compare images of a plurality of surrogate PET images.

In an embodiment, the PET imaging data can be PET list-mode data. PET list-mode data can be PET data acquired by event-by-event data acquisition. PET list-mode data acquisition with positron emission tomography can be advantageous over conventional frame mode data acquisition in terms of one or more of higher efficiency of data storage, higher temporal resolution, and/or higher flexibility of data manipulation and/or reconstruction. However, it should be understood that the underlying idea is not limited thereto and can be used with sinogram data instead of list-mode data. For example, subsets could then be chosen as when performing OSEM reconstruction with sinogram data.

Dividing the PET imaging data into subsets can comprise generating subsets by random selection of counts of the PET imaging data. For example, PET list-mode data can be used as an input. Subsets can be generated using random sub-sampling or random bootstrapping. An advantage can be a computationally efficient and fast generation of subsets.

In addition or in the alternative, dividing the PET imaging data into subsets can comprise chronologically dividing the PET imaging data into subsets, in particular generating subsets of chronologically overlapping PET imaging data. The PET imaging data may be divided into overlapping or non-overlapping subsets of chronological PET imaging data. For example, subsets may be generated using time-frame picking. A first subset may for example include events between event numbers 0 and 4M, a second subset may include events between 1M and 5M, etc. An optional overlap can reduce noise, because a higher number of counts may be included in each sub-set. The chronological dividing may help to reveal effects that evolve over time such as an uptake of a tracer that evolves over time.

In a further refinement, the device can be adapted to perform the step of identifying one or more local regions with extended dynamics, in particular based on said surrogate PET images reconstructed based on the chronologically divided PET imaging data. An advantage can be that effects may become apparent that would otherwise be obscured or hidden when image reconstruction was performed on the entire PET imaging data.

In an embodiment, the device can be adapted to perform the steps of: generating first subsets by random selection of counts of the PET imaging data and constructing a first set of first surrogate PET images based on the first subsets, wherein a first surrogate PET image is reconstructed for each of the first subsets; and generating second subsets by chronologically dividing the PET imaging data into second subsets, in particular generating second subsets of chronologically overlapping PET imaging data, and constructing a second set of second surrogate PET images based on the second subsets, wherein a second surrogate PET image is reconstructed for each of the second subsets; and comparing the first surrogate PET images from the first subsets and the second surrogate PET images from the second subsets. This allows to compare data from randomly generated surrogate PET images with data from subsets of chronologically overlapping PET imaging data, so that a differing behavior and potentially obscured temporal effects such as an uptake of a tracer that evolves over time or slight patient movements may be recognized.

The device can be adapted to (a) determine a (spatial) mean tracer concentration based on the surrogate PET images or based on a PET image reconstructed using the PET imaging data; and to (b) determine the expected variance based on said mean tracer concentration. A mean tracer concentration may be determined by taking an average over a plurality of the surrogate PET images. For example for one or more spatial positions averaging may be performed over the plurality of surrogate PET images. A spatial position can correspond to a pixel/voxel or group of pixels/voxels. A spatial, in particular a voxel wise, estimation for the mean tracer concentration can be determined over the plurality of surrogate PET images. The expected variance can be determined based thereon for example using a suitable statistical model. The statistical model may be selected depending on a statistical distribution of the observed process. In has been found that in PET imaging, the voxel noise can be modelled in good agreement using Poisson statistics. In addition or in the alternative, the mean tracer concentration may be determined based on a PET image reconstructed using the (entire) PET imaging data. Using a conventionally reconstructed PET image from the entire PET imaging data may also provide averaging so as to obtain an estimate of the mean tracer concentration to be expected over the surrogate PET images. The expected variance can again be determined based thereon.

The device can be adapted to perform a voxel-wise determination of the variance between images of the set of surrogate PET images and/or a voxel-wise determination of the confidence level measure based on a voxel-wise expected variance and a voxel-wise determined variance between images of the set of surrogate PET images. As used herein, the term voxel wise means that the determination of the variance and/or confidence is performed for a spatial or volumetric region of a voxel or group of voxels.

The device can be adapted to perform the step of evaluating in what (spatial) percentage of the reconstructed images each region exceeds a predefined threshold for providing a second confidence level measure. This allows to provide an additional confidence level measure for observed discrepancies. For example, in those voxels where the regularization has shown inconsistent behavior throughout the subsets (i.e. noise realizations), high uncertainty values may be observed. Depending on local noise in an image, a regularization may decide whether a strong filter should be applied, or even whether no filtering may be necessary.

The device can be adapted to (randomly) select a plurality of subsets of the surrogate PET images; for each of said selected subsets of the surrogate PET images performing the steps of: determining a variance between images of the subset of surrogate PET images; and determining a confidence level measure based on an expected variance and the determined variance between images of the subset of surrogate PET images; and generating a heat map indicative of a number of times the confidence level measure determined for each of the subsets of the surrogate PET images exceeds a predetermined threshold. The heat map can then be presented to a physician as an indicator regarding the reliability of image features representing true features of underlying physiological processes or merely representing artifacts or risk of having false-negatives.

The device can further comprise an output adapted to provide a graphical representation of the confidence level measure, in particular as a graphical overlay to at least one of a PET image, an MR image and/or CT image. The output can be a display device adapted to display the graphical representation of the confidence level measure either separately or together with a reconstructed PET image. The PET image may be a complete PET image reconstructed based on the complete (full count) PET imaging data. In addition or in the alternative, the output can be a data interface adapted to provide the graphical representation as data to be visualized by an already existing display or a computer screen. The graphical output may be represented for example as a variance map. In a refinement, the graphical representation can be a variance difference map indicative of a difference between the expected variance and the determined variance between images of the set of surrogate PET images. The graphical representation may allow to draw attention to suspicious regions or as on-demand information. Optionally, the determined confidence level measure may be used to alter a presentation of the PET image reconstructed from the original (full-count) list-mode or sinogram data set.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a system comprising a device for processing PET imaging data;
Fig. 2 shows an exemplary flow-chart of a method for processing PET imaging data;
Fig. 3 shows an exemplary flow-chart of a further refined method for processing PET imaging data;
Fig. 4 shows an exemplary flow-chart of a further refined method for processing PET imaging data;
Fig. 5 shows an exemplary PET image;
Fig. 6 shows an exemplary PET image with an overlay of confidence level measures;

Fig. 7 shows several PET images for difference confidence levels;
Fig. 8 shows a PET image, an overlay PET image and a confidence level map.

## DETAILED DESCRIPTION OF THE INVENTION

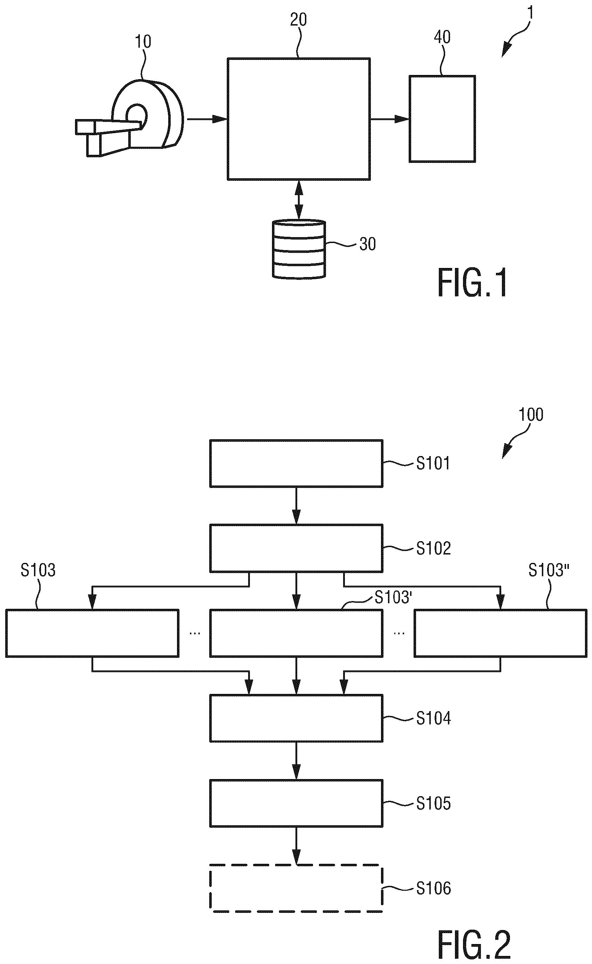

Fig. 1 shows an embodiment of a positron emission tomography system comprising a device for processing PET imaging data. The system is therein denoted in its entirety with reference numeral 1. The device for processing PET imaging data is denoted with reference numeral 20. The device 20 can be adapted to perform the processing steps as described in more detail with reference to Fig. 2. The system 1 can comprise a positron emission tomography, PET, scanner 10 adapted to acquire PET imaging data. The PET scanner can be an emission imaging device such a positron emission tomography device, a single photon emission computed tomography (SPECT) device or the like. In addition or in the alternative, a storage 30 such as a database comprising PET imaging data can be provided. The device 20 can be configured to obtain, i.e. to receive or to retrieve, the PET imaging data from the PET scanner 10 and/or from the storage 30 (e.g., an electronic medical record (EMR) database, a picture archiving and communication system (PACS) database, and the like). The system 1 or device 20 can further comprise an output 40, for example a display. The device 20 can be implemented using a computer or workstation or other electronic data processing device that is adapted to perform the steps as described in more detail further below. The electronic data processing device may comprise one or more typical components, such as at least one electronic processor, at least one user input device (e.g., a mouse, a keyboard, a trackball, and/or the like), and an output 40 such as a display device.

Fig. 2 shows an exemplary flow-chart of a method 100 for processing PET imaging data. Accordingly, Fig. 2 illustrates the steps that the device 20 of Fig. 1 is adapted to perform. In a first step S101, PET imaging data is obtained. The PET imaging data can be obtained directly from a PET scanner 10 or from a storage 30 as illustrated in Fig. 1. In a second step S102, the PET imaging data is divided into subsets. For example, the dividing the PET imaging data into subsets can comprise generating subsets by random selection of counts of the PET imaging data or by chronologically dividing the PET imaging data into subsets, in particular generating subsets of chronologically overlapping PET imaging data. Each subset of the PET imaging data comprises fewer counts than the PET imaging data obtained in step S101. In addition (not shown), the obtained PET imaging data may be processing in a conventional fashion to obtain a conventional PET image. Advantageously, the PET imaging data can be PET list-mode data, which allows computationally efficient processing and separation into subsets. In steps S103, S103', S103", ... the respective subsets of the PET imaging data are used to reconstruct a set of surrogate PET images based on the subsets, wherein a surrogate PET image is reconstructed for each of the subsets. This separate reconstruction of separate surrogate PET images is illustrated by the plurality of separate steps S103, S103', S103".

In step S104, a variance between images of the set of surrogate PET images is determined. For a (preferably each) spatial location the variance is determined over the set of surrogate PET images. The variance between images may thus be determined by evaluating a value of the set of the surrogate PET images at this spatial location. The value in each surrogate PET image represents an activity of a radioactive tracer which may be given as a count number or count rate. The variance over the set of surrogate PET images thus represents a statistical characteristic at this particular spatial location. The spatial location can correspond to a voxel or group of neighboring voxels.

In steps S105, a confidence level measure is determined based on an expected variance and the determined variance between images of the set of surrogate PET images. The confidence level measure can be provided separately for one or more different spatial locations in the image. The confidence level measure can be determined by comparing the determined variance between images of the set of surrogate PET images with an expected variance. For example, a mean count number or count rate at a spatial location may be determined based the surrogate PET images and the expected variance for this mean count number or count rate may be determined using a suitable statistical model such as Poisson statistics.

In an optional step S106, a graphical representation of the confidence level measure may be provided to a user such as a treating physician. For example, the graphical representation may be provided via an output 40 as illustrated in Fig. 1, in particular as a graphical overlay to a PET and/or CT image.

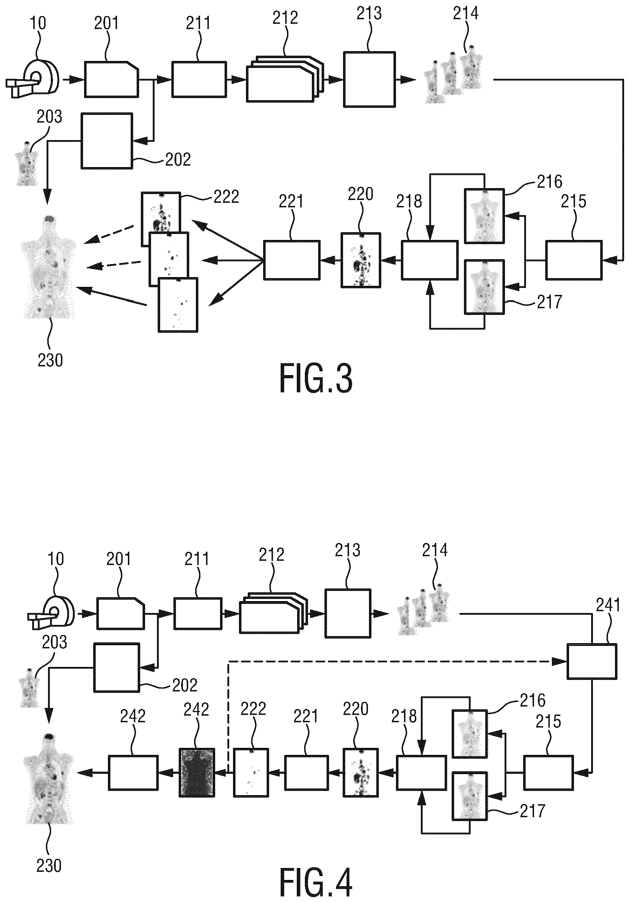

Fig. 3 shows an exemplary flow-chart of a further refined method for processing PET imaging data. The PET imaging data 201 can again be acquired by a PET scanner 10. For example, list-mode data can be acquired using an application-specific workflow or protocol. The obtained PET imaging data 201 can be processed in step 202 to reconstruct a PET image 203 indicative of a 3D activity distribution. Conventional image reconstruction can be applied in step 202.

Further, the PET imaging data 201 is used as an input to step 211, wherein the PET imaging data 201 is divided into subsets 212. For example, a set of Nb list-mode subsets can be created by random sub-sampling (case I) or by time frame picking (case II), each containing Pb% of the original acquisition, optionally such that Nb*Pb >= 100. Reconstructing these subsets in step 213 results in a set of surrogate PET images 214 (PB_1 ... PB_Nb). In step 215, for one or more voxels of the surrogate PET images, in particular for each voxel of PB_i, a statistical mean 217 and/or (absolute) variance 216 can be estimated. A simple way to calculate the mean is by taking the average of a voxel at location (x,y,z) over the entire set of all the Nb surrogate PET images. Another way one would be to also include one or more neighborhood voxels (x+/-dx,y+/-dy ,z+/-dz) of each PET image PB_i into this calculation. Depending on the size of Nb, Bessel's correction can be applied in the standard variance estimation in order to compensate for a sample size bias.

In step 218, the estimated spatial measures for mean (MEAN) and/or variance such as the absolute variance (VAR_abs) can be used to derive a 3D relative variance difference map 220. In order to calculate a deviation range, e.g. the average local standard variation can be estimated from the mean estimated in step 217 using a suitable statistical model. In PET imaging, the voxel noise can be modelled with good agreement using Poisson statistics. It is also possible to estimate the mean based on the image 203 reconstructed using all available data. Accordingly, a first estimate for the absolute value to be expected can be given by the (estimated) mean value itself. More advanced models, such as models including e.g. the related impact of spatial representation using BLOBs to this statistics, can also be considered in this step. Finally, the 3D relative variance difference map 220 (RVDM) can calculated from these measures, e.g. using the following formula:

$$RVDM = \begin{cases} (VAR\_abs\_measured - VAR\_abs\_modell) / MEAN; & MEAN > EPS \\ 0; & MEAN <= EPS \end{cases}$$

The case discrimination is optional. EPS may represent a small number preventing division-by-zero effects especially in an area outside the body, where zero mean values may occur. The relative variance difference between the determined variance and the expected variance can serve as a confidence level measure. The confidence level measure can be indicative of how well the determined variance between images of the set of surrogate PET images corresponds to a statistically expected variance.

In an optional further step 221, a thresholding operation may be performed on the variance difference map 220 to obtain several images 222 indicative of values of the variance difference map 220 exceeding one more predetermined thresholds.

In step 230, the result can be used to indicate deviations from the expected variance range to the user (i) as additional side-by-side image information and/or (ii) as an overlay to the original PET image 203. Optionally, a set of predetermined or user-selected threshold values 222 can be applied to spot/highlight areas outside a certain tolerance range. In addition or in the alternative, the result can be displayed as information on demand, for example when the user clicks on / hoovers over a certain area of interest of the reconstructed PET image 203.

In addition or in the alternative, the result may be used to adapt an intra-iterative regularization applied to reconstruct a PET image, for example by modifying one or more spatial prior characteristics. It will be appreciated that step 202 may thus be applied after determining the confidence level measure according to aspects of the present disclosure. It is also possible to reconstruct the PET image 203 in step 202 based on the original PET imaging data 201 in a first step and to reconstruct a further PET image 203' in a further step 202' (not shown) further taking into account the confidence level measure according to aspects of the present disclosure. This allows to perform a before/after-comparison.

In addition or in the alternative, the result may be used to alter a previously calculated PET image 203 in one or more post-processing and/or post-filtering steps, for example by adapting/providing additional spatial information fed into an artificial neural network applied in state-of-the-art noise regularization methods.

###

Fig. 4 shows an exemplary flow-chart of a further refined method for processing PET imaging data. The overall configuration and processing steps may be similar to the ones described with reference to Fig. 3. The following will thus focus on differences and additional aspects. Further to the features described with reference to Fig. 3, a loop can be introduced as indicated by the dashed line after box 222 to box 241. It is suggested that the processing further comprises a feedback loop adapted to derive a confidence measure based on the relative variance difference map 220, RVDM, or more generally based on an expected variance and the determined variance between images of the set of surrogate PET images. Instead of calculating the voxel variance and mean from the entire set of surrogate PET images, only a sub-set, in particular a random sub-set, of those may be selected in step 241. Binary, feature-thresholded variance difference maps 222 may calculated for respective sample sets and the spatial result can be added to a 3D feature heat map 242. Subsequently, the process is re-started on a new set constellation for a predefined number of repetitions, as indicated by the dashed arrow from item box 222 to box 241 in Fig. 4. Using the resulting heat map 242, the resulting confidence level measure determined in step 243 may be defined as the number of times a spatial feature exceeded the feature threshold value divided by the number of random set constellations tested. The resulting heat map can be used to either derive additional information displayed to the user and/or to directly refine the PET image 203 to be displayed as an imaging result 230, for example on a display 40 as indicated in Fig. 1.

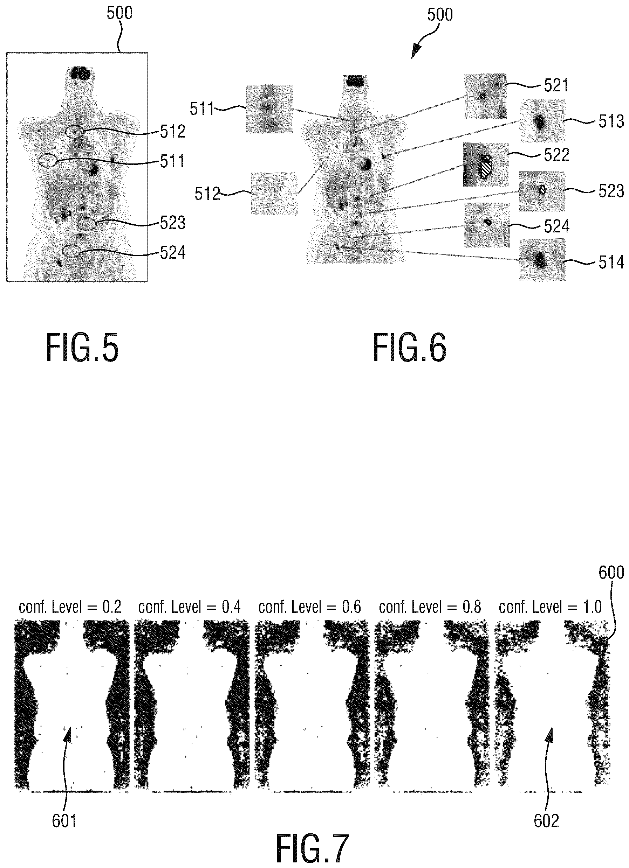

Fig. 5 and Fig. 6 show an exemplary PET image comprising with an overlay to provide a graphical representation of the confidence level measure. The figures show results from applying the method as described with reference to Fig. 3 to a test patient with suspicious findings. Fig. 5 shows a reference image for a clinical dataset reconstructed using 100% of list-mode data using an intra-iterative regularized reconstruction setup. In this figure, some areas are highlighted which may be considered potential lesions in a former visual assessment. By applying the method described with reference to Fig. 3, using 64 samples of 25% bootstrapping subsets and a feature threshold of 1, the result in Fig. 6 was achieved. It shows a clear spatial correlation of extended variation in many of these spots. The portions wherein the confidence level measure exceeds a predetermined threshold are indicated in 521, 522, 523 and 524 as shaded areas. Other highly evident lesions indicated by 511, 512, 513, 514 such as the one in the right pelvis or the left rib cage and the potential lesion at the middle of the right rib cage did not show any relevant indication. The proposed approach can thus further assist a clinician in determining relevant findings.

Fig. 7 shows several PET images for different confidence levels. The figure shows the experimental result 600 for applying the method as described with reference to Fig. 4, wherein different confidence level thresholds may be applied in step 221 and/or step 243. As can be seen from the left map in Fig. 7, a low confidence level threshold may show a higher sensitivity such that a higher number of features 601 is highlighted. If a high confidence level threshold is selected as exemplarily illustrated in the right map in Fig. 7, the number of features 602 is reduced such that only features exceeding the confidence level threshold are highlighted.

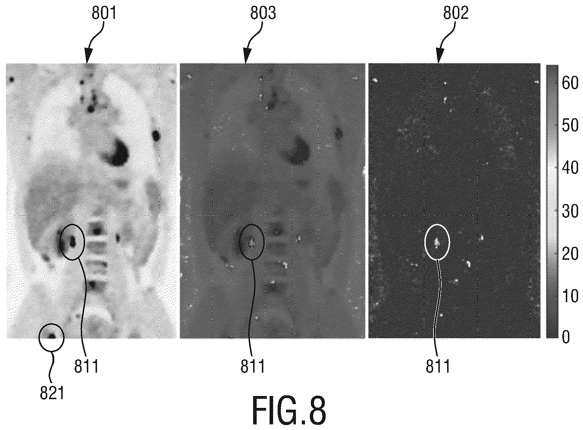

Fig. 8 shows a PET image 801, an overlay PET image 803 and a confidence level map 802. The overlay PET image 803 is based on a combination of the PET image 801 and the confidence level map 802. For example, the confidence level can be a heat map as constructed in step 242 of Fig. 4. As illustrated in Fig. 8, image features 811 and 821 may show a similar value in the PET image 801. The value can be indicative of a metabolic activity of a radioactive tracer. However, as can be seen from the confidence level map 802 and the overlay 803, the feature 811 indicates an uncertainty based on an expected variance and the determined variance between images of the set of surrogate PET images. Accordingly, there is a certain probability that the feature 811 may not be a finding with the same relevance as the feature 821 even though they appear similar in the PET image 801.

It should be noted that optional pre-processing steps may be applied to the PET imaging data. For example, it may not be necessary to use data from the entire imaging volume or to use the entire acquired data. A conceivable variation is that in order to determine a (local) variance and an associated confidence level measure it is not necessarily necessary to reconstruct the complete PET volume. Instead, pre-processing of the list-mode data and the reconstruction can be limited to a certain area within this volume, which may speed up the evaluation considerably.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device (20) for processing PET imaging data, wherein the device is adapted to perform the steps of:

   - obtaining PET imaging data (201) (S101);
   - dividing the PET imaging data into subsets (212) (S102);
   - reconstructing a set of surrogate PET images (214) based on the subsets (212) (S103), wherein a surrogate PET image is reconstructed for each of the subsets;
   - determining a variance (216) between images of the set of surrogate PET images (S104);
   - determining a confidence level measure (220, 222) based on an expected variance and the determined variance between images of the set of surrogate PET images (S105).

2. Device (20) according to claim 1, wherein dividing the PET imaging data (201) into subsets (212) comprises dividing the measured counts of the PET imaging data into a number of subsets, each subset having fewer counts than the measured counts of the PET imaging data.

3. Device (20) according to claim 1, wherein the PET imaging data is PET list-mode data.

4. Device (20) according to any of the preceding claims, wherein dividing the PET imaging data (201) into subsets (212) comprises generating subsets by random selection of counts of the PET imaging data.

5. Device (20) according to any of the preceding claims, wherein dividing the PET imaging data (201) into subsets (212) comprises chronologically dividing the PET imaging data into subsets, in particular generating subsets of chronologically overlapping PET imaging data.

6. Device (20) according to claim 5, wherein the device is adapted to perform the step of the step of identifying one or more local regions with extended dynamics based on said surrogate PET images reconstructed based on the chronologically divided PET imaging data.

7. Device (20) according to claims 4 and 5 or 6, wherein the device is adapted to perform the steps of:

   - generating first subsets by random selection of counts of the PET imaging data and constructing a first set of first surrogate PET images based on the first subsets, wherein a first surrogate PET image is reconstructed for each of the first subsets; and
   - generating second subsets by chronologically dividing the PET imaging data into second subsets, in particular generating second subsets of chronologically overlapping PET imaging data, and constructing a second set of second surrogate PET images based on the second subsets, wherein a second surrogate PET image is reconstructed for each of the second subsets; and
   - comparing the first surrogate PET images from the first subsets and the second surrogate PET images from the second subsets.

8. Device (20) according to any of the preceding claims, wherein the device is adapted to (a) determine a mean tracer concentration based on the surrogate PET images or based on a PET image reconstructed using the PET imaging data; and to (b) determine the expected variance based on said mean tracer concentration.

9. Device (20) according to any of the preceding claims, wherein the device is adapted to perform a voxel-wise determination of the variance between images of the set of surrogate PET images and/or a voxel-wise determination of the confidence level measure based on a voxel wise expected variance and the voxel-wise determined variance between images of the set of surrogate PET images.

10. Device (20) according to any of the preceding claims, further adapted to perform the step of evaluating in what percentage of the reconstructed images each region exceeds a predefined threshold for providing a second confidence level measure.

11. Device (20) according to any of the preceding claims, wherein the device is adapted to

   - select a plurality of subsets of the surrogate PET images (241);
   - for each of the subsets of the surrogate PET images performing the steps of

     - determining a variance between images of the subset of surrogate PET images; and
     - determining a confidence level measure based on an expected variance and the determined variance between images of the subset of surrogate PET images; and

   - generating a heat map (242) indicative of a number of times the confidence level measure determined for each of the subsets of the surrogate PET images exceeds a predetermined threshold.

12. Device according to any of the preceding claims, further comprising an output (40) adapted to provide a graphical representation of the confidence level measure, in particular as a graphical overlay to at least one of a PET image, an MR image, and/or CT image.

13. Positron emission tomography, PET, system (1) comprising:

   - a positron emission tomography, PET, scanner (10) adapted to acquire PET imaging data; and
   - a device (20) for processing PET imaging data according to any of the preceding claims.

14. Method (100) for processing PET imaging data comprising the steps of:

   - obtaining PET imaging data(201) (S101);
   - dividing the PET imaging data into subsets(212) (S102);
   - reconstructing a set of surrogate PET images (214) based on the subsets (212) (S103), wherein a surrogate PET image is reconstructed for each of the subsets;
   - determining a variance (216) between images of the set of surrogate PET images (S104);
   - determining a confidence level measure (220, 222) based on an expected variance and the determined variance between images of the set of surrogate PET images (S105).

15. Computer program product comprising program code means for causing a computer to carry out the steps of the method (100) as claimed in claim 14 when said computer program is carried out on a computer.

FIG.1

FIG.2

FIG.3

FIG.4

FIG.5

FIG.6

conf. Level = 0.2    conf. Level = 0.4    conf. Level = 0.6    conf. Level = 0.8    conf. Level = 1.0

FIG.7

FIG.8

Europäisches
Patentamt

European
Patent Office

Office européen
des brevets

# EUROPEAN SEARCH REPORT

**Application Number**

EP 22 19 3386

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/270443 A1 (VIJA ALEXANDER HANS [US]) 18 September 2014 (2014-09-18) | 1-4,8-15 | INV.<br>A61B6/03<br>A61B6/00<br>G06T11/00 |
| Y | * paragraphs [0018], [0052] - [0054]; figures 1,2 * | 5,6 | |
| Y | MARKIEWICZ P J ET AL: "Rapid processing of PET list-mode data for efficient uncertainty estimation and data analysis", PHYSICS IN MEDICINE AND BIOLOGY, INSTITUTE OF PHYSICS PUBLISHING, BRISTOL GB, vol. 61, no. 13, 9 June 2016 (2016-06-09), XP020306418, ISSN: 0031-9155, DOI: 10.1088/0031-9155/61/13/N322 [retrieved on 2016-06-09] | 5,6 | |
| A | * Section 2.7 "Dynamic projection view analysis" * | 1-4,7-15 | |
| X | MARKIEWICZ P J ET AL: "Assessment of bootstrap resampling performance for PET data", PHYSICS IN MEDICINE AND BIOLOGY, INSTITUTE OF PHYSICS PUBLISHING, BRISTOL GB, vol. 60, no. 1, 9 December 2014 (2014-12-09), pages 279-299, XP020276000, ISSN: 0031-9155, DOI: 10.1088/0031-9155/60/1/279 [retrieved on 2014-12-09] | 1-4, 8-10, 13-15 | TECHNICAL FIELDS SEARCHED (IPC)<br><br>A61B<br>G06T |
| A | * Section 2.3.1 "World of multiple realizations" Section 2.4 "Processing and analysing datasets"; figures 2,3 * | 5-7,11, 12 | |
| A | PRIOR ART PUBLISHING, 24 February 2017 (2017-02-24), XP040687130, * the whole document * | 1-15 | |

The present search report has been drawn up for all claims

| Place of search | Date of completion of the search | Examiner |
|---|---|---|
| Munich | 21 November 2022 | Marzal-Abarca, X |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 3386

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2022

| Patent document cited in search report | Publication date | Patent family member(s) | Publication date |
|---|---|---|---|
| US 2014270443 A1 | 18-09-2014 | NONE | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018220182 A1